# EUROPEAN PATENT SPECIFICATION

(11) **EP 2 286 887 B1**
(45) Date of publication and mention of the grant of the patent: **25.01.2012**
(21) Application number: 10160979.0
(22) Date of filing: 26.04.2010
(51) Int. Cl.: B01D 15/36, B01J 41/00, C07C 231/24

(54) **Endotoxin removal in contrast media**
Entfernung von Endotoxin in Kontrastmitteln
Élimination d'endotoxine dans un milieu de contraste

(30) Priority: 21.07.2009 US 227107 P
(43) Date of publication of application: 23.02.2011
(73) Proprietor: GE Healthcare AS, 0401 Oslo (NO)
(72) Inventor: Hagelin, Gunnar, 0401 Oslo (NO); Wistrand, Lars-Göran, 0401 Oslo (NO)
(74) Representative: Wulff, Marianne Weiby

(56) References cited:
- US-A- 5 550 287
- US-A1- 2005 211 621
- US-B1- 6 432 306
- GULBRANDSEN T: "ION EXCHANGE IN THE PROCESSING OF NON-IONIC X-RAY CONTRAST MEDIA" CAPLUS, 1982, pages V-36-V-43, XP001203585 Oslo Symposium
- ANSPACH F B ET AL: "Removal of endotoxins by affinity sorbents" JOURNAL OF CHROMATOGRAPHY, ELSEVIER SCIENCE PUBLISHERS B.V, NL LNKD- DOI:10.1016/0021-9673(95)00126-8, vol. 711, no. 1, 8 September 1995 (1995-09-08), pages 81-92, XP004038953 ISSN: 0021-9673

## Description

### CROSS REFERENCE TO RELATED APPLICATIONS

The present application claims benefit of priority under 35 U.S.C. §119(e) to United States Provisional Application number 61/227,107 filed July 21, 2009.

### TECHNICAL FIELD

This invention relates to the manufacture of contrast media, and more specifically to the removal of endotoxins in such contrast media.

### BACKGROUND OF THE INVENTION

A general problem in the production of various contrast media relates to the presence of endotoxins which are produced by bacterial proliferation during the handling of large quantities of aqueous solutions. While the bacteria themselves are easily removed or destroyed in various sterilization procedures, endotoxins are at times left intact.

Endotoxins have been known and studied for many years, particularly in regard to the pathophysiological reactions in animals. For many years it was believed that endotoxin material was contained within gram-negative bacilli cells and was released only upon disintegration of the cell walls. Hence, the material was termed endotoxin. Recent studies, however, suggest that endotoxin is localized at the cell surface of gram-negative bacilli and may be present with viable and killed cells as well as in a free form within a liquid media.

Endotoxins are known to cause several and varied pathophysiological reactions and have been identified as direct and contributory causes of death of many hospitalized patients. Endotoxins are known to cause febrile reactions in animals with symptoms of extremely high fever, vasodilation, diarrhea, and the like and, in extreme cases, fatal shock. It is also known that endotoxins cause leucocytosis, deleterious changes in carbohydrate and protein metabolism and widespread intravascular clotting by fibrin formation.

Since contrast media are often injected into hospital patients in large quantities, the contrast media must first be purified of endotoxin contamination.

Common methods for removal of endotoxins from solutions are ultra-filtration, adsorptive matrices as chromatographic resins and ion exchange resins. Several problems have been identified with these methods such as clogging of filters, substantial loss of substance and difficult, costly and time consuming post treatment.

US 5972225 describes a method of removing endotoxins from bulk, non-ionic iodinated contrast media comprising dissolving the media in an aqueous starting solution and passing the solution through a filtration zone containing activated carbon.

It is a need for fast and cost-effective ways to remove endotoxins in contrast media. More specifically, there is a need to develop such method that gives endotoxin values of less than about 1 EU/ml with minimal effect on the concentration of the contrast agent, the content of different cations, the pH and ionic strength of the formulation.

### SUMMARY OF THE INVENTION

The present invention provides a method for the purification of formulated contrast media by removal of endotoxins by using filtration with anion exchange membranes.

Thus, viewed from one aspect, the invention provides a method for removing endotoxins from formulated contrast media comprising passing said media through an anion exchange membrane.

The method according to the invention is simple to perform, highly efficient and has negligible impact on the pH of and the other parameters of the formulation.

### DETAILED DESCRIPTION OF THE INVENTION

Generally, the manufacture of contrast media involves the production of the chemical drug substance (referred to as primary production) followed by formulation into the drug product (referred to as secondary production).

A method is provided for removing endotoxins from such contrast media. In this regard, the term "contrast media" as used herein is intended to mean any substance used to enhance the contrast of structures or fluids within the body in medical imaging, except negatively charged ionic contrast media. In the method of the present invention, the contrast media is a product after formulation in the secondary production.

An important problem with some methods for endotoxin removal according to prior art is that the purification process can not handle formulated contrast media with high viscosity. Even more importantly, endotoxins cannot be removed without also altering the composition of the contrast media.

To solve this problem and also to fulfill above mentioned criteria, the present invention provides a method for removing endotoxins from formulated contrast media comprising passing said media through an anion exchange membrane.

The formulated contrast media used according to the present invention can preferably be contrast media for magnetic resonance imaging (MRI), for example Gd-based media, or for X-ray imaging.

Preferred X-ray contrast media used according to the present invention are non-ionic iodinated contrast media. More preferably said contrast media are highly viscous, preferably with viscosities of about 20-35 mPas.

More preferably the non-ionic contrast media comprises 1,3-Bis(formylamino)-N,N'-bis[3,5-bis(2,3-dihydroxypropylaminocarbonyl)-2,4,6-triiodophenyl]-2-hydroxy-propane. Most preferably the non-ionic contrast media is Visipaque™, which is one of the most used media in diagnostic X-ray procedures. Iodixanol is the non-proprietory name of the chemical drug substance of Visipaque™.

The solution to be treated in accordance with the present invention will advantageously have the iodinated drug substance present at a concentration sufficient to provide between about 50 milligrams and 500 milligrams of organically bound iodine per milliliter of solution (mg l/ml), more preferably about 100-400 mg/l ml.

Further, in accordance with the invention, the solution prior to purification will normally contain endotoxins at a level exceeding 0.2 EU per 50 mg l (0.2 EU/50 mg l), and often exceeding about 5 EU/50 mg l.

The anion exchange membrane provides effective, rapid removal of endotoxins from the contrast media at hand. The separation occurs as a result of the selective exchange of ions in the contrast media with counter ions in the membrane. The contrast media exhibits essentially no affinity for the membrane, and thus the endotoxin is effectively separated from contrast media solutions. The negatively charged endotoxin will adsorb to the positively charged groups on the ion exchanger and thus be removed from the solution. When the exchanger is equilibrated with the same ionic strength of buffer used for formulation, one negative buffer counter ion will be exchanged for each endotoxin molecule adsorbed.

In an industrial scale the solution will preferably pass through the anion exchange membrane at a rate that will optimize not only the amount of endotoxin that is removed from the solution passing through the membrane, but also the volume of solution that is passed through the membrane in a given amount of time. Flow rates of between 1 and 10 l/min., more preferably between 2 and 5 l/min., will be typical for processes of the invention.

The system will preferably be operated at a pressure of between 6894 Pa and 689475 Pa (1 psig and 100 psig) more preferably between 34473 Pa and 103421 Pa (5 psig and 15 psig), most preferably about 68947 Pa (10 psig). Further, the system will maintain a solution temperature that will allow for the desired separation and which is below the decomposition temperature of the drug substance involved. Preferably, processes of the invention will be operated at a solution temperature at between about room temperature and about 4° C.

The purification system will desirably be allowed to run within the above parameters for the duration sufficient to achieve the desired reduction of endotoxin to acceptable levels. Typically, such runs will last between about 0.5 and about 10.0 hours, more preferably between about 1.5 and about 2.5 hours. Samples can be periodically taken and tested to monitor endotoxin levels, and the process can be discontinued once acceptable levels are achieved.

The end product will be comprised of a solution of contrast media containing essentially all of the total contrast media of the starting solution. The product solution will contain the endotoxin at a substantially reduced level as compared to the starting solution, and will more preferably be essentially free from endotoxin (i.e. containing endotoxin at a level of less than about 1 EU/ml).

The invention is illustrated further by the following examples that are not to be construed as limiting the invention in scope to the specific procedures or products described in them.

As can be seen from example 1, the method of the present invention achieved a reduction in endotoxin level to less than 1 EU/ml in a relatively short, commercially-feasible period of time. At the same time the potency of Visipaque™ and Prohance™ remained unchanged. Processes of the invention thus provide for extremely cost-effective means for reducing endotoxin contamination in contrast media.

Example 2 shows the removal of endotoxins from a solution comprising 1,3-Bis(formylamino)-N,N'-bis[3,5-bis(2,3-dihydroxypropylaminocarbonyl)-2,4,6-triiodophenyl]-2-hydroxy-propane. It can be seen that the level of endotoxins decreases dramatically to a level of less than 0.5 EU/ml, and importantly also one can see that the concentration of sodium ions and calcium ions in the solution are essentially unchanged.

### EXAMPLES

### EXAMPLE 1

### Samples.

*a)* The iodinated aromatic X-ray compound Visipaque™ (Iodixanol 320 mg iodine/ml in 10 mM TRIS buffer pH 7.4, osmolality adjusted to 290 mmol/kg with sterile, endotoxin free NaCl, viscosity = 24 mPa).
*b)* The Gd-chelate based contrast medium for MR Prohance™ (Gd-DOTA, 0.8 M in 10 mM TRIS buffer pH 7.4, osmolality adjusted to 290 mmol/kg with sterile, endotoxin free NaCl).

### Preparation of stock solution of endotoxin from E. Coli.

One vial (10 mg, 10 e 7 EU, Sigma Corp., USA) was added 9 ml water for injection (WFI) and 1 ml acetonitrile to give a concentration of 9.1 e6 EU/ml = 9.1 e3 EU/µl.

*Preparation of working solution of LPS from E. Coli:* 0.1 ml of the stock solution was diluted to 10 ml with WFI (dilution factor = 100) to give 91 EU/µl.

### Spiking of formulations with endotoxin.

To 10 ml aliquots of Visipaque™ and Prohance™ were added 3 and 6 µl working solution of LPS from *E.coli* to a final theoretical concentration of 270 and 550 EU endotoxin respectively.

### Depyrogenation procedure.

*a) Filter device:* Sartobind Q15™ membranes integrated in ready-to-use units with pore size 3-5 µm, made of cellulose modified with positively charged quaternary ammonium groups.
*b) Equilibration and sterilization of Sartobind*^{™}*Q15.*
   Each filter was eluted with 50 ml 10 mM TRIS pH 7.4, followed by 5 ml 70% ethanol that was allowed to stay in the filter for 3 minutes and finally with 50 ml 10 mM TRIS pH 7.4 (sterile).
*c*) *Endotoxin removal.*
   The formulations were passed through the filter units by a flow of about 1-2 drops per second.

### Sampling for endotoxin analysis.

Due to the high viscosity, the formulation was mixed thoroughly after elution through the Sartobind™ Q15 anion exchanger before sampling to endotoxin analysis.

### Analysis of endotoxin content

The method used for determination of the endotoxin content, was the chromogenic method described in the US and European pharmacopeia with a sensitivity of 0.005 EU/ml. 1:200 dilution were prepared by diluting 50 µl formulation in 9.95 ml WFI (Water for Injection) and the sample analyzed on the kinetic QCL instrument together with a positive control. If the positive control was positive, the test was considered valid and the results were automatically calculated by the software. No endotoxin present would give a result of NMT 1 EU/ml.

Result values of endotoxin, pH and concentration of iodine and gadolinium are shown in Table 1. Note that the values of endotoxin are the measured ones, and differ from the theoretical values given in the experimental section. * Reference value is 80.4 mg Gd/ml; ** Reference value is 320 mg l/ml.

**Table 1**

| **Sample** | **Endotoxin Pre filtration** **(EU/ml)** | **Endotoxin post filtration** **(EU/ml)** | **pH of formulation** | **Gd-content** **(mg/ml)** | **Iodine content** **(mg I/ml)** |
|---|---|---|---|---|---|
| **Visipaque** **320 mg I/ml** | 220 | < 0.5 | 7.2 | - | 326** |
| **Visipaque** **320 mg I/ml** | 460 | < 0.5 | 7.3 | - | 318** |
| **Prohance** **80.4 mgGd/ml** | 185 | < 0.5 | 7.2 | 80.0* | - |
| **Prohance** **80.4 mgGd/ml** | 417 | < 0.5 | 7.2 | 78.9* | - |

### EXAMPLE 2

Samples of a solution comprising 1,3-Bis(formylamino)-N,N'-bis[3,5-bis(2,3-dihydroxypropylaminocarbonyl)-2,4,6-triiodophenyl]-2-hydroxy-propane were prepared by dissolving the pure compound in sterile water containing 10 mM tris buffer and 0.1 mg/ml CaNa₂EDTA to a concentration of ca 320 mg l/ml. Calcium chloride was added to the concentrations given below and the osmolality of the solutions were adjusted to isotonicity (290 mOsm/kg) by the addition of sodium chloride. Finally, the solutions were sterilized by autoclavation at 120 °C for 30 minutes. The samples were then prepared to contain endotoxins and purified as in Example 1 with the exception that a Sartobind Q100^{™} was used. The endotoxin levels and the Ca²⁺ and Na⁺ levels before and after purification are shown in Table 2.

**Table 2**

| **Sample** | **Endotoxin Pre filtration** **(EU/ml)** | **Endotoxin post filtration** **(EU/ml)** | **Ca²⁺ Pre filtration** **(mM)** | **Ca²⁺ Post filtration** **(mM)** | **Na⁺ Pre filtration** **(mM)** | **Na⁺ Post filtration** **(mM)** |
|---|---|---|---|---|---|---|
| 1 | 14.80 | 0.3 | 0.368 | 0.353 | 44.5 | 45.1 |
| 2 | 19.55 | 0.7 | 0.570 | 0.545 | 45.5 | 44.6 |
| 3 | 17.29 | 1.4 | 0.946 | 0.926 | 44.7 | 43.8 |

## Claims

1. Method for removing endotoxins from formulated contrast media comprising passing said media through an anion exchange membrane.

2. Method as claimed in claim 1 wherein the contrast media is a non-ionic iodinated contrast media.

3. Method as claimed in claim 1 wherein the contrast media have viscosities of about 20-35 mPas.

4. Method as claimed in claim 1 wherein the contrast media comprises 1,3-Bis(formylamino)-N,N'-bis[3,5-bis(2,3-dihydroxypropylaminocarbonyl)-2,4,6-triiodophenyl]-2-hydroxy-propane.

## Patentansprüche

1. Verfahren zur Entfernung von Endotoxinen aus formulierten Kontrastmitteln, umfassend das Leiten der Mittel durch eine Anionenaustauschmembran.

2. Verfahren nach Anspruch 1, wobei das Kontrastmittel ein nichtionisches iodiertes Kontrastmittel ist.

3. Verfahren nach Anspruch 1, wobei die Kontrastmittel Viskositäten von etwa 20 bis 35 mPas aufweisen.

4. Verfahren nach Anspruch 1, wobei das Kontrastmittel 1,3-Bis(formylamino)-N, N'-bis[3,5-bis(2,3-dihydroxypropylaminocarbonyl)-2,4,6-triiodphenyl]-2-hydroxy-propan umfasst.

## Revendications

1. Procédé d'élimination d'endotoxines d'un milieu de contraste formulé comprenant le passage dudit milieu à travers une membrane d'échange d'anion.

2. Procédé selon la revendication 1 dans lequel le milieu de contraste est un milieu de contraste non ionique iodé.

3. Procédé selon la revendication 1 dans lequel le milieu de contraste présente une viscosité de 20 à 35 m.Pa.s environ.

4. Procédé selon la revendication 1 dans lequel le milieu de contraste comprend du 1,3-Bis(formylamino)-N,N'-bis[3,5-bis(2,3-dihydroxypropylaminocarbonyl)-2,4,6-tri-iodophényl]-2-hydroxy-propane.
